# EUROPÄISCHE PATENTSCHRIFT

(11) **EP 1 434 637 B1**
(45) Veröffentlichungstag und Bekanntmachung des Hinweises auf die Patenterteilung: **08.02.2006**
(21) Anmeldenummer: 02774618.9
(22) Anmeldetag: 14.09.2002
(51) Int. Cl.: B01D 1/00

(54) **VERFAHREN UND TRENNMODUL ZUM ABTRENNEN VON PARTIKELN AUS EINER DISPERSION, INSBESONDERE VON BLUTKÖRPERCHEN AUS BLUT**
METHOD AND SEPARATING MODULE FOR THE SEPARATION OF PARTICLES FROM A DISPERSION, IN PARTICULAR OF BLOOD CORPUSCLES FROM BLOOD
PROCEDE ET MODULE DE SEPARATION POUR SEPARER DES PARTICULES D'UNE DISPERSION, NOTAMMENT POUR SEPARER DES CORPUSCULES SANGUINS CONTENU DANS LE SANG

(30) Priorität: 12.10.2001 DE 10150549
(43) Veröffentlichungstag der Anmeldung: 07.07.2004
(73) Patentinhaber: Roche Diagnostics GmbH, 68305 Mannheim (DE); F. HOFFMANN-LA ROCHE AG, 4070 Basel (CH)
(72) Erfinder: EFFENHAUSER, Carlo, 69469 Weinheim (DE); OCVIRK, Gregor, 68239 Mannheim (DE); FIEDLER, Wolfgang, 69514 Laudenbach (DE)
(74) Vertreter: Pfeifer, Hans-Peter
(86) Internationale Anmeldenummer: PCT/EP2002/010336
(87) Internationale Veröffentlichungsnummer: WO 2003/033096

(56) Entgegenhaltungen:
- EP-A- 0 057 907
- WO-A-99/09042
- US-A- 5 922 210
- PATENT ABSTRACTS OF JAPAN vol. 10, no. 57 (P-434) [2114], 7. März 1986 (1986-03-07) & JP 60 201253 A (NIPPON DENSHI K.K.), 11. Oktober 1985 (1985-10-11)
- PRIES A.R.; SECOMB T.W.; GAEHTGENS P.: 'Biophysical aspects of blood flow in the microvasculature' CARDIOVASCULAR RESEARCH Bd. 32, 1996, ELSEVIAR, Seiten 654 - 667

## Beschreibung

Die Erfindung betrifft ein Verfahren zum Abtrennen von Partikeln aus einer Dispersion und ein Bauteil zur Durchführung eines solchen Verfahrens. Da ein solches Bauteil modular als Bestandteil unterschiedlicher Systeme verwendet werden kann, wird es hier als Trennmodul bezeichnet. Die Erfindung kann insbesondere zum Abtrennen von korpuskulären Bestandteilen aus biologischen Proben, vor allem aus Blut verwendet werden.

Auf verschiedenen Anwendungsgebieten stellt sich das Problem, aus einer Dispersion, die in einem Trägermedium dispergierte Partikel enthält, die Partikel teilweise oder vollständig zu entfernen. Ein besonders wichtiges Gebiet sind analytische Verfahren zur Bestimmung der Konzentration von Bestandteilen im Blut. Solche Bluttests können in vielen Fällen nicht mit Vollblut durchgeführt werden, das die korpuskulären Bestandteile (Blutkörperchen) enthält. Vielmehr ist es notwendig, zuvor aus dem Vollblut Plasma zu gewinnen, das möglichst frei von zellulärem Material ist.

Die Erfindung eignet sich jedoch auch zur Behandlung anderer Dispersionen, wobei das Trägermedium nicht nur flüssig, sondern auch gasförmig sein kann. Ein Beispiel für die Anwendung der Erfindung im Rahmen diagnostischanalytischer Verfahren, bei dem eine nichtbiologische Flüssigkeit behandelt wird, ist die Manipulation, Anreicherung oder Isolation von sogenannten beads, die aufgrund ihrer großen erneuerbaren Oberfläche in jüngerer Zeit beispielsweise in der kombinatorischen Chemie und der Molekularbiologie verstärkt verwendet werden. Darüber hinaus kann die Erfindung auch auf anderen Gebieten der chemischen Verfahrenstechnik und der Lebensmittelindustrie zum Einsatz kommen, um Partikel aus Prozeßströmen zu separieren. Weitere Nutzungsmöglichkeiten bestehen bei biotechnologischen Verfahren (Entfernung und Isolierung von Zellkulturen aus entsprechenden Dispersionen) sowie auf dem Gebiet der Abwasserreinigung. Ohne Beschränkung der Allgemeinheit wird nachfolgend auf die Behandlung von Dispersionen in Flüssigkeiten, hauptsächlich auf die Abtrennung von Plasma aus Vollblut, Bezug genommen.

Traditionell wurden Zentrifugationsverfahren eingesetzt, um durch Abtrennen der zellulären Bestandteile Plasma für Bluttests zu gewinnen. Sie eignen sich jedoch nicht für moderne miniaturisierte Tests. Dies gilt insbesondere für die sogenannte patientennahe Diagnostik, bei der ein möglichst kleines und kompaktes Analyseelement (beispielsweise in Form eines Teststreifens) alle für die Durchführung des Tests notwendigen Reagenzien und sonstigen Mittel enthält, so daß nur noch die Probenflüssigkeit in Kontakt mit dem Analyseelement gebracht werden muß, um nach kurzer Zeit anhand einer physikalisch an dem Analyseelement nachweisbaren Veränderung (insbesondere einer Farbänderung oder einer Änderung einer elektrischen Meßgröße) das gewünschte analytische Resultat visuell oder mit Hilfe eines Auswertegerätes zu bestimmen.

Um für derartige Tests aus relativ kleinen Blutvolumina Plasma zu gewinnen, werden seit vielen Jahren Filtrationsverfahren diskutiert und teilweise auch mit Erfolg verwendet, bei denen unterschiedliche Filtermedien, insbesondere mikroporöse Membranen und Glasfaservliese, zum Einsatz kommen. Frühe Beispiele dieser Filtrationstechniken sind in den US-Patentschriften 3,791,933 und 4,477,575 beschrieben. Ein neueres Beispiel mit einer aufwendigen Kombination aus Membran- und Glasfaserfiltern ist Gegenstand des US-Patentes 6,045,699.

In dem US-Patent 5,922,210 ist ein Mikrobauteil beschrieben, das dazu dienen soll, extrem kleine Plasmamengen im Bereich bis etwa 1 *µ*l durch Mikrofiltration zu gewinnen. Dabei werden in einem Siliziumsubstrat durch Ätzen Mikrokanäle erzeugt. Die Abtrennung der Blutkörperchen erfolgt in einem sogenannten Sperrkanal (barrier channel) dessen Tiefe weniger als 0,1 *µ*m beträgt, so daß die Blutkörperchen nicht durch den Sperrkanal hindurchfließen können. Die erforderlichen Zuflußkanäle und der Sperrkanal werden in zwei aufeinanderfolgenden Herstellungsschritten erzeugt. Die erforderliche, extrem geringe Tiefe des Sperrkanals von weniger als 0,1 µm wird durch die Dauer des Ätzvorgangs in einem Ätzbad bestimmt. Im Hinblick auf die erforderliche hohe Reproduzierbarkeit ist dieser Herstellungsprozeß sehr schwierig und aufwendig.

Die vorbekannten Plasmagewinnungsmethoden haben erhebliche Nachteile. Vor allem besteht ein hohes Risiko, daß die feinen Poren durch mechanischen Verschluß oder durch Adhäsion von zellulärem Material an den Porenwänden verstopft werden. Dadurch wird die Filterkapazität begrenzt.

Eine Vergrößerung der Filterkapazität bedingt einen größeren Raumbedarf des Filtermediums. Außerdem ist die Relation zwischen aufgebrachtem Probenvolumen und gewonnenem Plasmavolumen ungünstig. Schließlich können durch Adhäsion von Proteinen an dem Filtermedium oder durch die beim Durchtritt von Erythrozyten durch die engen Filterporen auftretenden hohen Scherkräfte und die daraus resultierende Hämolyse Meßfehler verursacht werden.

Hiervon ausgehend liegt der Erfindung die Aufgabe zugrunde, die Abtrennung von Partikeln aus einer Dispersion unter möglichst weitgehender Vermeidung der vorstehend geschilderten Nachteile mit einem Trennmodul zu ermöglichen, das einfach und kostengünstig hergestellt werden kann. Das Trennmodul soll vorzugsweise ein zur einmaligen Verwendung vorgesehenes sogenanntes "Disposable" sein und sich insbesondere zur Erzeugung kleiner Plasmamengen (weniger als 10 *µ*l, insbesondere weniger als 5 *µ*l) für miniaturisierte Tests eignen.

Die Aufgabe wird gelöst durch ein Verfahren zum Abtrennen von Partikeln aus einer fluiden Dispersion, insbesondere zum Abtrennen von korpuskulären Bestandteilen aus biologischen Proben, vor allem aus Blut, mittels eines Trennmoduls mit einem Substrat mit Strömungskanälen in Form von nutenförmigen Vertiefungen in einer Oberfläche des Substrats, umfassend einen Zuflußkanal zum Zuführen der Dispersion zu einer Verzweigung, einen ersten Abflußkanal zum Ableiten von Fluid mit verminderter Partikelkonzentration von der Verzweigung weg und einen zweiten Abflußkanal zum Ableiten von Fluid mit erhöhter Partikelkonzentration von der Verzweigung weg, wobei das Fluid in dem zweiten Abflußkanal so viel schneller als in dem ersten Abflußkanal strömt, daß die dispergierten Partikel an der Verzweigung aufgrund der unterschiedlichen Strömungsgeschwindigkeit bevorzugt in dem zweiten Abflußkanal weiterströmen.

Die bisher für die Zwecke der Erfindung verwendeten Filtrationsverfahren basieren auf sterischer Selektion, also darauf, daß die abzutrennenden Partikel zurückgehalten werden, weil die Poren des Filtermediums kleiner als der Durchmesser der Teilchen sind. Um auf diese Weise Erythrozyten zuverlässig abzutrennen, darf der Porendurchmesser des Filtermediums (insbesondere wegen der leichten Deformierbarkeit der Erythrozyten) höchstens etwa 1 *µ*m betragen.

Bei der Erfindung basiert die Selektion auf einem völlig anderem Prinzip: Unterschiedliche lokale Teilchenströmungsgeschwindigkeiten in verschiedenen Strompfaden des Flüssigkeitsstroms in dem Trennmodul führen zu Schubspannungen, die bewirken, daß die Partikel an der Verzweigung bevorzugt in den zweiten Abflußkanal mit der höheren Strömungsgeschwindigkeit weiterströmen. Der erste Abflußkanal mit der geringeren Strömungsgeschwindigkeit enthält eine verminderte Partikelkonzentration.

Durch die Erfindung werden eine Vielzahl wichtiger Vorteile erreicht:
- Da die Abtrennung der Partikel nicht auf einer sterischen Selektion basiert, kann die kleinste Dimension der Abflußkanäle größer als der Partikeldurchmesser sein. Beispielsweise haben die Strömungskanäle eines für die Plasmagewinnung aus Vollblut geeigneten Trennmoduls vorzugsweise eine kleinste Querschnittsdimension von mindestens 5 µm und höchstens 150 µm, wobei Werte von weniger als 100 µm, insbesondere weniger als 50 µm besonders bevorzugt sind. Damit besteht im Gegensatz zu den vorbekannten Filtrationsverfahren praktisch kein Risiko der Verstopfung eines Filtermediums. Dieser Vorteil wird noch dadurch verstärkt, daß keinerlei fasrige Materialien verwendet werden müssen, die zusätzliche Verstopfungsrisiken bergen.
- Erfindungsgemäß kann Blut (oder eine andere Dispersion) kontinuierlich über lange Zeiträume behandelt werden. Das Trennmodul kann deshalb für die kontinuierliche Gewinnung (praktisch) partikelfreier Filtrate oder auch für die kontinuierliche Partikelanreicherung aus Dispersionen eingesetzt werden.
- Die Fertigung ist relativ einfach und zu günstigen Kosten möglich. Im Vergleich zu vorbekannten Filtrationsverfahren entfällt die Fertigung und Integration eines Filtermediums in das Trennmodul. Im Vergleich zu dem in dem US-Patent 5,922,210 beschriebenen Mikrofilter ist die Fertigung wesentlich einfacher, weil die in dem Chip integrierten Strömungskanäle vergleichsweise große Dimensionen haben. Derartige Kanalstrukturen können kostengünstig in Großserien hergestellt werden. Insbesondere eignet sich ein Verfahren, bei dem zunächst auf photolithographischem Wege ein Master hergestellt wird. Von diesem Master läßt sich eine Form gewinnen, mit der wiederum Produktchips durch Pressen oder Spritzgießen hergestellt werden (Beispiel: Herstellung von CDs). Kleinere Stückzahlen können durch Laserablation produziert werden.
- Für die Herstellung ist vorteilhaft, daß die Erfindung keine unterschiedlich tiefen Strukturen erfordert. Vorzugsweise sind mindestens beide Abflußkanäle, besonders bevorzugt sämtliche Strömungskanäle, gleich tief. Sie können auf einfache Weise in einem einzigen Arbeitsgang hergestellt werden.
- Das in den Strömungskanälen des Trennmoduls befindliche Totvolumen ist sehr gering. Die Erfindung ermöglicht es deshalb, aus einem sehr kleinen Probenvolumen ein ausreichend großes Volumen an Plasma zu gewinnen.
- Das erfindungsgemäße Trennmodul kann weitergehend miniaturisiert werden als ein System, das ein Filtermedium und Ableitungskanäle enthält, ohne daß sich dadurch die Effizienz der Trennung oder der Durchsatz verringern. Auch dies reduziert die Kosten.
- Das Trennmodul kann einfach in ein System, insbesondere ein Analysesystem integriert werden. Im Rahmen analytischer Mikrosysteme besteht beispielsweise die Möglichkeit einer "planaren Integration", wobei für die Analyse notwendige Reagenzien und Flüssigkeitsbehandlungselemente in den gleichen Chip integriert werden, in dem sich die Strömungskanäle des Trennmoduls befinden. Es ist jedoch auch eine konventionelle Ankopplung an ein Analysesystem über Schlauchleitungen mit geringem Totvolumen möglich.

Die der Erfindung zugrundeliegenden physikalischen Effekte lassen sich teilweise auf Basis von experimentellen Untersuchungen des Strömungsverhaltens von Blut im Kapillarsystem des Körpers und hierauf basierenden theoretischen Überlegungen erklären. Die vorliegenden Erkenntnisse sind beispielsweise in einem Review-Artikel von A. R. Pries et al. "Biophysical aspects of blood flow in the microvasculature", Cardiovascular Research 32, 1996, 654-667 zusammengefaßt. Dort wird unter anderem berichtet, daß an Verzweigungen der das Blut im Körper transportierenden Kapillargefäße der Hämatocrit (Gehalt an roten Blutkörperchen) in einem Tochtergefäß mit geringerem-Blutstrom in der Regel niedriger als in einem Tochtergefäß mit höherem Blutstrom ist. Es wird erörtert, daß sich diese Phasenseparation wegen der zahlreichen Einflußgrö-ßen und der in mehrerlei Hinsicht nichtlinearen Abhängigkeit des Blutstromes von diesen Einflußgrößen nur unzureichend theoretisch beschreiben läßt. Im einzelnen werden der "plasma skimming effect", der "network Fahraeus effect" und der "pathway effect" als physikalische Prinzipien, die die Phasenseparation in kapillaren Blutgefäßen bestimmen, diskutiert. Einer dieser Effekte, nämlich der network Fahraeus effect, beschreibt die Tendenz roter Blutkörperchen, an einer Verzweigung bevorzugt dem Strömungsweg mit der höheren Flußrate (und damit zusammenhängend der höheren Strömungsgeschwindigkeit) zu folgen.

Nach dem Kenntnisstand der Erfinder ist davon auszugehen, daß dieses Prinzip die Funktion des erfindungsgemäßen Trennmoduls im wesentlichen erklärt. Allerdings konnte nicht erwartet werden, daß tatsächlich eine nahezu vollständige Plasmaseparation mit einfach realisierbaren Mitteln in einem praktisch nutzbaren Umfang erreicht werden könnte. Dies wird auch dadurch bestätigt, daß die grundsätzlichen Kenntnisse über die Phasenseparation an Kapillarverzweigungen schon sehr lange bekannt sind. Beispielsweise werden in dem zitierten Review-Artikel experimentelle In-Vitro-Untersuchungen von 1964 und In-Vivo-Studien von 1970 zitiert.

Die Eignung dieses Prinzips zur Plasmaseparation war auch deshalb nicht zu erwarten, weil in den natürlichen Kapillaren keine weitgehende oder sogar vollständige Trennung zu beobachten ist. Im Gegenteil ist die Funktion des menschlichen Körpers davon abhängig, daß auch in den feinsten Kapillaren noch eine so hohe Konzentration an Erythrozyten vorhanden ist, daß die Sauerstoffversorgung gesichert ist. Die Verhältnisse sind auch insofern grundsätzlich verschieden, als im lebenden Körper ein Netzwerk aus Adern mit elastischen Wänden mit im Rhythmus des Blutpulses stark schwankenden Strömungsgeschwindigkeiten durchströmt wird, während die Flüssigkeit in einem Trennmodul mit konstanter Geschwindigkeit zwischen starren Wänden strömt.

Den Publikationen über das Strömungsverhalten in Blutkapillaren ist selbstverständlich keinerlei Hinweis darüber zu entnehmen, daß und auf welche Weise ein für praktische Zwecke brauchbares Trennmodul hergestellt werden könnte. Von besonderer Bedeutung für den praktischen Erfolg der Erfindung ist eine bevorzugte Ausführungsform, gemäß der die Tiefe mindestens des Zuflußkanals, bevorzugt auch des ersten Abflußkanals und besonders bevorzugt sämtlicher Strömungskanäle, mindestens auf deren unmittelbar an die Verzweigung angrenzenden Kanalabschnitt größer als die Kanalbreite ist. Diese bevorzugte Ausführungsform hängt mit der Tatsache zusammen, daß die Funktion hinsichtlich der Abtrennung der Partikel im wesentlichen durch die Breite der Kanäle in der unmittelbaren Nachbarschaft der Verzweigung bestimmt ist. Durch eine im Verhältnis zu der Breite große Tiefe kann die Trennleistung (pro Zeiteinheit getrenntes Flüssigkeitsvolumen) ohne Beeinträchtigung der Funktion erhöht werden.

Die Erfindung wird nachfolgend anhand von in den Figuren dargestellten Ausführungsbeispielen näher erläutert. Die dargestellten und beschriebenen Besonderheiten können einzeln oder in Kombination eingesetzt werden, um bevorzugte Ausgestaltungen der Erfindung zu schaffen. Es zeigen:
- Fig. 1: eine schematische perspektivische Darstellung einer ersten Ausführungsform eines erfindungsgemäßen Trennmoduls
- Fig. 2-4: nicht maßstäbliche Querschnittsdarstellungen entlang den Schnittlinien A bis C von Fig. 1
- Fig. 5: eine schematische zeichnerische Wiedergabe des an einer Verzweigung mittels Videodarstellung visuell beobachtbaren Trenneffektes
- Fig. 6: eine graphische Darstellung der Abhängigkeit des Trenneffektes von der Relation der lokalen Strömungsgeschwindigkeit in den Abflußkanälen
- Fig. 7: eine schematische perspektivische Darstellung einer zweiten Ausführungsform eines erfindungsgemäßen Trennmoduls
- Fig. 8: und 9 nicht maßstäbliche Querschnittsdarstellungen entlang den Schnittlinien A und B von Fig. 7
- Fig. 10: eine nicht maßstäbliche perspektivische Darstellung einer dritten Ausführungsform eines erfindungsgemäßen Trennmoduls und
- Fig. 11: eine schematische Aufsicht auf ein Analyseelement mit einer planaren Integration eines erfindungsgemäßen Trennmoduls.

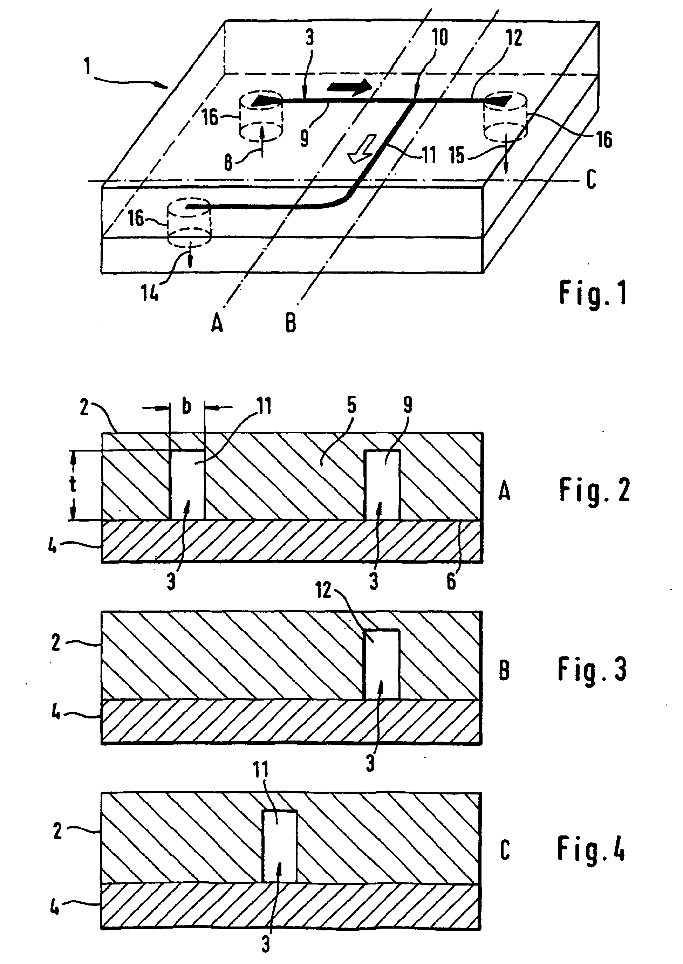

Das in den Figuren 1 bis 4 dargestellte Trennmodul 1 besteht im wesentlichen aus einem Kanalteil 2 mit Strömungskanälen 3 und einem Deckelteil 4. Bei der Herstellung des Kanalteils 2 werden in einem scheibenförmigen Substrat 5, beispielsweise mittels eines der oben erwähnten Verfahren, die Strömungskanäle 3 in Form mikroskopisch kleiner nutenförmiger Vertiefungen in einer Oberfläche 6 des Substrat 5 erzeugt.

Die Kanäle sind in den Figuren, vor allem in den Figuren 2 und 4 in stark übertriebener Größe dargestellt. Typischerweise liegt ihre Breite b unter 150 µm, wobei sich speziell für die Plasmagewinnung insbesondere Kanalbreiten von weniger als 100 *µ*m, bevorzugt weniger als 50 *µ*m, bewährt haben. Andererseits liegen die bevorzugten Dimensionen so weit über dem Wellenlängenbereich von sichtbarem Licht, daß die erforderlichen Strukturen in der Oberfläche 6 des Substrats 5 problemlos mittels photolithographischer Verfahren erzeugt werden können, wie sie von der Herstellung elektronischer Chips bekannt sind. Bevorzugt beträgt die Breite b der Kanäle mindestens etwa 5 µm.

Über einen Einlaß 8 wird Vollblut (oder eine andere Dispersion, aus der Partikel abgetrennt werden sollen) in das Trennmodul 1 eingespeist und über einen Zuflußkanal 9 einer Verzweigung 10 zugeführt, an der sich der Flüssigkeitsstrom in einen ersten Abflußkanal 11 und in einen zweiten Abflußkanal 12 verzweigt. Die in den Abflußkanälen 11 und 12 strömende Flüssigkeit wird über Auslässe 14 bzw. 15 aus dem Trennmodul 1 entnommen. Die Ein- bzw. Auslässe werden im dargestellten Fall von in dem Deckelteil 4 vorhandenen Bohrlöchern 16 gebildet, an die geeignete Leitungen, wie beispielsweise Kunststoffschläuche angeschlossen werden können.

Für die Erfindung ist wesentlich, daß die Flüssigkeit in dem zweiten Abflußkanal 12 soviel schneller als in dem ersten Abflußkanal 11 strömt, daß an der Verzweigung 10 die dispergierten Partikel aufgrund der unterschiedlichen Strömungsgeschwindigkeit bevorzugt in dem zweiten Abflußkanal weiterströmen.

Im Falle der Behandlung von Blut enthält der erste Abflußkanal 11 Plasma mit einer (in Abhängigkeit von den Verfahrensbedingungen) mehr oder weniger geringen Restkonzentration an Blutkörperchen. Er wird deshalb nachfolgend als Plasmakanal bezeichnet. Der zweite Kanal 12 (mit der höheren Strömungsgeschwindigkeit) enthält eine im Vergleich zum Ausgangsblut erhöhte Konzentration an Blutkörperchen. Da diese Flüssigkeit für analytische Zwecke nicht gebraucht wird, wird er nachfolgend als Waste-Kanal bezeichnet. Diese Kurzbezeichnungen dürfen aber nicht als Beschränkung des Anwendungsgebietes der Erfindung verstanden werden.
- Zum einen muß der "Plasmakanal" nicht reines Plasma enthalten. Bei der Erprobung der Erfindung wurde festgestellt, daß eine einzige Verzweigung ausreichen kann, um ein für analytische Zwecke ausreichend reines "analytisches Plasma" zu gewinnen. Die in dem ersten Abflußkanal strömende Flüssigkeit enthält jedoch in der Regel eine geringe Restkonzentration an Blutkörperchen.
- Zum zweiten gibt es Anwendungsgebiete der Erfindung, bei denen der Zweck der Abtrennung der Partikel nicht (wie bei der Plasmagewinnung) in der Reinigung der Trägerflüssigkeit der Dispersion liegt, sondern das Ziel ein Konzentrat der dispergierten Partikel ist. In diesem Fall enthält der erste Abflußkanal mit der höheren Strömungsgeschwindigkeit (der im Falle der Plasmagewinnung den Waste-Kanal bildet) nicht einen Abfall (waste), sondern das gewünschte Produkt.

Die Strömungsgeschwindigkeiten in den Abflußkanälen 11 und 12, ihre Relation zueinander und der damit verbundene Trenneffekt werden von einer Mehrzahl von Einflußfaktoren bestimmt, die sich in folgende Gruppen einteilen lassen:
a) Strömungswiderstand
   Wenn alle anderen Einflußfaktoren für beide Abflußkanäle 11, 12 gleich sind, ist die mittlere Durchflußrate umgekehrt proportional zu dem Strömungswiderstand der Kanäle.
b) Druckverhältnisse an den Ein- und Auslässen
   Der Druck an dem Einlaß 8 beeinflußt die Strömungsgeschwindigkeit in den Abflußkanälen 11, 12 (bei gleichen Druckverhältnissen an den Auslässen 14, 15) im wesentlichen proportional ohne ihre Relation zueinander zu verändern. Hingegen können unterschiedliche Druckverhältnisse an den Auslässen einen großen Einfluß auf die Relation der Strömungsgeschwindigkeiten haben.
   Wenn es bei einem bestimmten Anwendungsfall technisch möglich und wirtschaftlich vertretbar ist, an mindestens einen der Auslässe 14, 15 eine Pumpe mit exakter Pumprate anzuschließen, kann die Strömungsgeschwindigkeit in den Abflußkanälen 11, 12 über diese Pumprate eingestellt werden. Im Falle miniaturisierter Analyseelemente ist der Anschluß einer Pumpe in der Regel nicht möglich oder zumindest zu aufwendig. In diesem Fall können jedoch die Druckverhältnisse an den Auslässen 14, 15 dadurch beeinflußt werden, daß sich dort Materialien befinden, die die Flüssigkeit durch Kapillarkräfte aufsaugen und dadurch die Strömung in dem vorausgehenden Abflußkanal beschleunigen oder einen zusätzlichen Strömungswiderstand bilden und dadurch die Strömungsgeschwindigkeit vermindern.
c) Viskosität der Flüssigkeit
   Wenn die Viskosität in den Strömungskanälen unterschiedlich ist, wird die Strömungsgeschwindigkeit hiervon beeinflußt. Beispielsweise ist im Falle der Plasmagewinnung die Viskosität der Flüssigkeit in dem Plasmakanal 11 niedriger als in dem Waste-Kanal 12.
   Dies führt - bei sonst gleichen Bedingungen - zu einer relativen Erhöhung der Strömungsgeschwindigkeit in dem Plasmakanal 11.
d) Geschwindigkeitsprofil über den Kanalquerschnitt
   Für den Trenneffekt ist nicht die mittlere Strömungsgeschwindigkeit der Flüssigkeit in den Strömungskanälen (Volumenstrom pro Querschnittfläche und pro Zeiteinheit) entscheidend, sondern das lokale Geschwindigkeitsprofil im Bereich der Verzweigung. Es hängt in komplizierter Weise von verschiedenen Einflußfaktoren, darunter der genauen Kanalgeometrie, dem Material der Kanalwände und der Viskosität der Flüssigkeit ab.

Wegen der Vielzahl dieser Einflußgrößen ist es nicht möglich, für alle Anwendungsfälle eine allgemeinverbindliche Regel für die Dimensionierung der Strömungskanäle 3 anzugeben. Sie muß im Einzelfall experimentell festgelegt werden. Dennoch können auf Basis der experimentellen Erprobung der Erfindung folgende Angaben über vorteilhafte Dimensionierungsregeln gemacht werden:

Wie bereits erwähnt sind die Tiefen t der Kanäle 9, 11, 12 mindestens in den an die Verzweigung 10 unmittelbar angrenzenden Kanalabschnitten größer als die Breite b. Vorzugsweise beträgt das Aspektverhältnis A (Verhältnis der Kanaltiefe t zu der Kanalbreite b: A = t/b) mindestens A = 3, vorzugsweise mindestens A = 5, besonders bevorzugt mindestens A = 7. Besonders bevorzugt ist eine Ausführungsform, bei der die Tiefen mindestens des Zuflußkanals 9 und des Plasmakanals 11 in dem jeweils unmittelbar an die Verzweigung 10 angrenzenden Kanalabschnitt gleich groß sind. Besonders bevorzugt gilt dies für alle an die Verzweigung angeschlossenen Kanäle 9, 11, 12.

Wie ebenfalls bereits erwähnt beträgt die kleinste Querschnittsdimension des Plasmakanals mindestens 5 *µ*m und höchstens 150 *µ*m, wobei Werte kleiner als 100 *µ*m, insbesondere kleiner als 50 *µ*m, besonders bevorzugt sind.

Der Strömungswiderstand des Plasmakanals 11 sollte in der Regel höher als der Strömungswiderstand des Waste-Kanals 12 sein. Bevorzugt wird dies zumindest teilweise dadurch bewirkt, daß der Plasmakanal länger als der Waste-Kanal ist. Dies ist vorteilhaft, weil sich der Strömungswiderstand der Abflußkanäle 11, 12 leichter und präziser durch eine entsprechende Einstellung von deren Länge als mittels einer entsprechenden Dimensionierung des Querschnitts einstellen läßt.

Im Hinblick auf eine einfache Herstellung und präzise Funktion ist es weiterhin vorteilhaft, wenn die Tiefe t der Abflußkanäle 11, 12 mindestens auf einem Teil ihrer Länge, bevorzugt auf ihrer gesamten Länge, gleich ist. Vorzugsweise stimmt auch die Tiefe des Zuflußkanals 9 mit der (gleichen) Tiefe der Abflußkanäle 11, 12 überein.

Die Strömungskanäle 3 sind vorzugsweise über nahezu ihre gesamte Länge gleich breit, wobei es sich aber als vorteilhaft erwiesen hat, wenn mindestens der Zuflußkanal 9 und der Waste-Kanal 12 im Bereich des Einlasses 8 beziehungsweise des Auslasses 15 so gestaltet sind, daß scharfe Ecken, durch die Erythrozyten beschädigt werden könnten, vermieden werden. In Fig. 1 ist in diesem Bereich ein schräger Verlauf der Wände der genannten Kanäle 9 und 12 angedeutet.

Im Hinblick auf eine einfache Herstellung ist es zweckmäßig, wenn mindestens die beiden Abflußkanäle 11, 12, vorzugsweise auch der Zuflußkanal gleich breit sind.

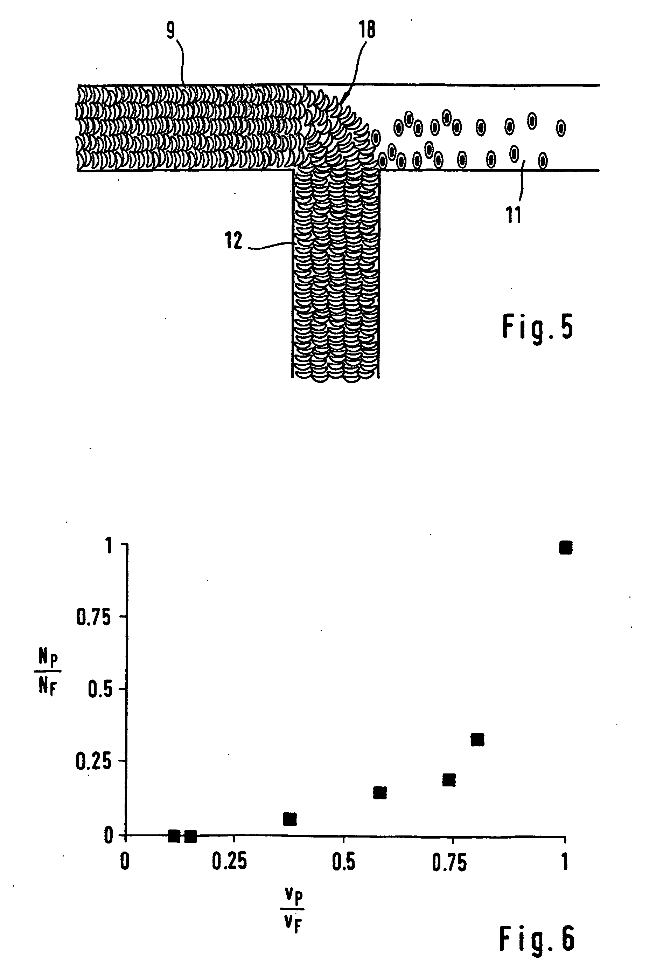

Im Rahmen der experimentellen Erprobung der Erfindung wurde das Strömungsverhalten von Erythrozyten mit Hilfe eines auf die Verzweigung gerichteten Mikroskops und Videoaufzeichnung beobachtet. Eine schematische zeichnerische Darstellung eines typischen Bildes zeigt Fig. 5. Abweichend von dem Trennmodul gemäß den Figuren 1 bis 4 wurde dabei eine Anordnung gewählt, bei der der Plasmakanal 11 in gerader Fortsetzung des Zuflußkanals 9 verläuft, während der Waste-Kanal 12 rechtwinklig von dieser Linie abzweigt. Die Darstellung zeigt, daß der der Erfindung zugrundeliegende Effekt im wesentlichen unabhängig davon ist, in welcher Richtung die Abflußkanäle 11, 12 von dem Zuflußkanal 9 abzweigen. Die Erythrozyten 18 folgen zum größten Teil dem Strompfad mit der größeren Strömungsgeschwindigkeit, obwohl sie dabei ihre Strömungsrichtung ändern müssen.

Fig. 6 zeigt eine graphische Darstellung experimenteller Daten, die die Abhängigkeit des Trenneffektes von der Relation der Strömungsgeschwindigkeit in den Abflußkanälen verdeutlicht. Sie wurden mit einem Trennmodul entsprechend Fig. 1 gewonnen, wobei der Zuflußkanal 9 und der Waste-Kanal 12 jeweils 32 µm breit und 32 µm tief waren. Der Plasmakanal 11 war 16 µm breit und 32 µm tief. Der Fluß pro Zeiteinheit in dem Zuflußkanal 9 lag im Bereich zwischen 0,01 und 0,5 µl/min. Auf der Abszisse ist die Relation der Strömungsgeschwindigkeit v_{P} in dem Plasmakanal 11 zu der Strömungsgeschwindigkeit v_{F} in dem Zuflußkanal (F für "feed channel") aufgetragen. Die Ordinate zeigt die entsprechende Relation der Teilchenzahlen N_{P} zu N_{F}. Die Experimente wurden mit im Verhältnis 1:5 verdünntem Blut durchgeführt, um die Erythrozyten besser erkennbar zu machen. Die Geschwindigkeiten v_{P} und v_{F} wurden aus der Videobeobachtung der im zentralen Strompfad strömenden Erythrozyten abgeleitet. Auch die Teilchenzahlen wurden aus den Videodaten bestimmt.

Man erkennt, daß der Trenneffekt mit abnehmendem Geschwindigkeitsverhältnis v_{P}/v_{F} rasch besser wird. Bei einem Geschwindigkeitsverhältnis von 0,75 strömen noch etwa 25 % der ursprünglichen Erythrozytenzahl in dem Plasmakanal. Wenn die Strömungsgeschwindigkeit im Plasmakanal weniger als etwa ein Viertel der Strömungsgeschwindigkeit im Zuflußkanal beträgt, wird eine ausgezeichnete Reinheit des Plasmas in dem Plasmakanal erreicht.

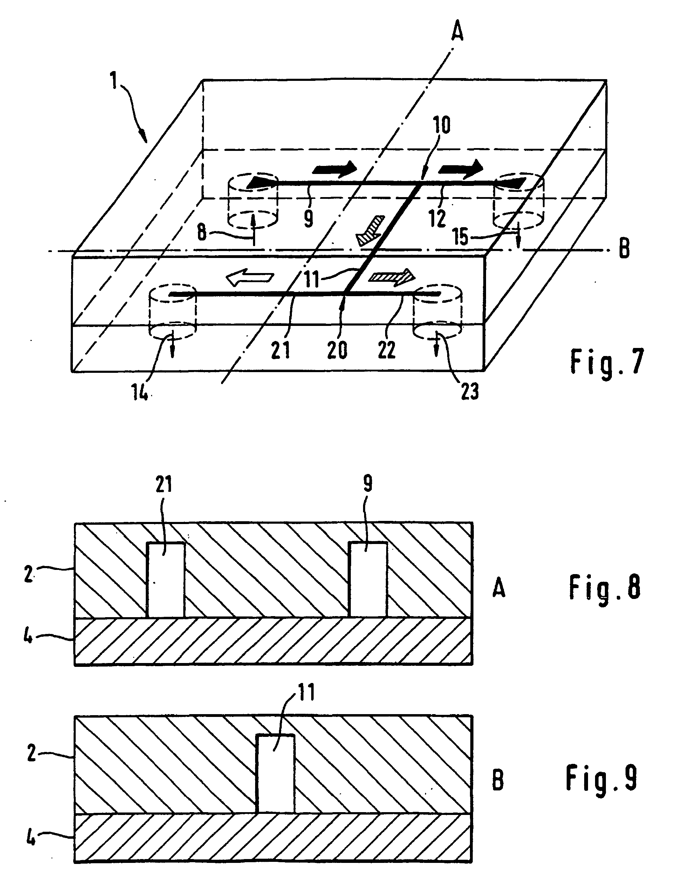

Die Figuren 7 bis 9 zeigen eine Ausführungsform eines Trennmoduls, bei dem in den Strömungskanälen, die von dem Einlaß 8 zu dem Plasma-Auslaß 14 führen zwei Verzweigungen 10 und 20 derartig hintereinander angeordnet sind, daß durch einen zweistufigen Trennprozeß der Gesamt-Trenneffekt verbessert wird. Dies läßt sich dadurch erreichen, daß - wie dargestellt - der von der ersten Verzweigung 10 ausgehende Plasmakanal zu einer weiteren Verzweigung 20 führt, so daß er für die weitere Verzweigung 20 einen Zuflußkanal bildet und von der weiteren Verzweigung 20 ein weiterer Plasmakanal 21 und ein weiterer Waste-Kanal 22 abzweigen, wobei der Plasmakanal 21 zu dem Plasmaauslaß 14 und der Waste-Kanal 22 zu einem zweiten Waste-Auslaß 23 führt. Auch in diesem Fall sind die Dimensionen der Abflußkanäle und die Betriebsbedingung so gewählt, daß die Flüssigkeit in dem weiteren Waste-Kanal 22 soviel schneller als in dem weiteren Plasmakanal 21 strömt, daß an der zweiten Verzweigung 20 eine Trennung in einem Flüssigkeitsstrom mit höherer Partikelkonzentration (Waste-Kanal 22) und in einem Flüssigkeitsstrom mit niedrigerer Partikelkonzentration (Plasmakanal 21) stattfindet.

Falls auch die mit einem zweistufigen Trennprozeß erreichbare Reinheit des Plasmas nicht ausreichen sollte, kann das Trennverfahren mittels eines entsprechend abgewandelten Trennmoduls auch drei- oder mehrstufig durchgeführt werden. Ein hierfür geeigneter (nicht dargestellter) Trennmodul weist eine Folge von Verzweigungen auf, deren jeweiliger Zuflußkanal von dem Plasmakanal der vorausgehenden Verzweigung gebildet wird, wobei an jeder dieser Verzweigungen die erläuterten Strömungsgeschwindigkeitsverhältnisse eingehalten werden.

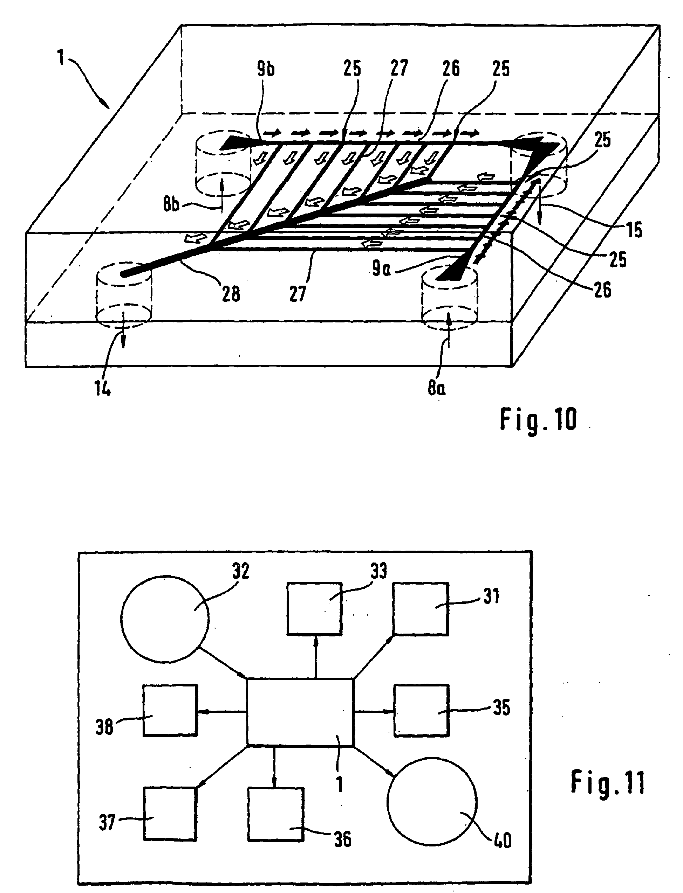

Fig. 10 zeigt eine Ausführungsform eines Trennmoduls 1, bei der eine erhöhte Trennleistung dadurch erreicht wird, daß eine Folge von Verzweigungen 25 derartig hintereinander angeordnet ist, daß jeweils der Waste-Kanal der vorausgehenden Verzweigung den Zuflußkanal der nachfolgenden Verzweigung bildet. Die von den Verzweigungen 25 abzweigenden Plasmakanäle 27 führen in eine gemeinsame Sammelleitung 28 und von dort zu dem Plasmaauslaß 14. Im dargestellten Fall ist eine derartige Anordnung symmetrisch zweifach vorhanden, wobei das Blut durch Einlässe 8a und 8b in den ersten Zuflußkanal 9a bzw. 9b eingespeist wird. Von dort strömt es längs eines Kanals 26a bzw. 26b, dessen zwischen den Verzweigungen 25 liegende Teilabschnitte jeweils den Waste-Kanal der vorausgehenden Verzweigung und den Zuflußkanal der nachfolgenden Verzweigung bilden. Der erfindungsgemäße Trenneffekt führt dazu, daß in diesen Kanälen 26a und 26b die Konzentration der Erythrozyten ständig zunimmt. Um dennoch eine gleichbleibende Plasmaqualität zu erreichen, sind die Plasmakanäle (im dargestellten Fall hinsichtlich ihrer Länge) so dimensioniert, daß die Strömungsgeschwindigkeit der darin transportierten Flüssigkeit in der Richtung abnimmt, in der die an der jeweiligen Verzweigung zugeführte Erythrozytenkonzentration zunimmt.

Fig. 11 zeigt eine mögliche Konzeption, gemäß der ein Trennmodul 1 gemeinsam mit anderen für eine Analyse erforderlichen modularen Elementen planar in einen Analysechip 31 integriert ist. Dabei ist der Einlaß des Trennmoduls 1 mit einem Blutreservoir 32 verbunden. An mehrere Auslässe des Trennmoduls 1 sind Testmodule 33 bis 38 angeschlossen, die zur Bestimmung unterschiedlicher Analyten oder beispielsweise auch zur genaueren Analyse unterschiedlicher Konzentrationsbereiche des gleichen Analyten dienen können. Schließlich ist in den Analysechip 31 ein Waste-Behälter 40 integriert, in den die Flüssigkeit aus einem oder mehreren Waste-Auslässen des Trennmoduls 1 geleitet wird.

Wie erwähnt wurde in den vorstehenden Erläuterungen lediglich beispielhaft und ohne Beschränkung der allgemeinen Anwendbarkeit der Erfindung auf die Abtrennung von Blutkörperchen aus Blut zur Gewinnung von Plasma Bezug genommen. Die Erläuterungen gelten sinngemäß auch für andere Anwendungsfälle, wobei in diesem Fall der Begriff "Plasmakanal" durch "erster Kanal", der Begriff "Waste-Kanal" durch "zweiter Kanal", der Begriff "Waste" durch "Flüssigkeit mit erhöhter Partikelkonzentration" und der Begriff "Plasma" durch "Flüssigkeit mit verminderter Partikelkonzentration" ersetzt werden müssen.

## Patentansprüche

1. Verfahren zum Abtrennen von Partikeln aus einer fluiden Dispersion, insbesondere zum Abtrennen von korpuskulären Bestandteilen aus biologischen Proben, vor allem aus Blut,
mittels eines Trennmoduls mit einem Substrat (5) mit Strömungskanälen (3) in Form von nutenförmigen Vertiefungen in einer Oberfläche (6) des Substrats (5), umfassend
- einen Zuflußkanal (9) zum Zuführen der Dispersion zu einer Verzweigung (10),
- einen ersten Abflußkanal (11) zum Ableiten von Fluid mit verminderter Partikelkonzentration von der Verzweigung (10) weg und
- einen zweiten Abflußkanal (12) zum Ableiten von Fluid mit erhöhter Partikelkonzentration von der Verzweigung (10) weg,
wobei das Fluid in dem zweiten Abflußkanal so viel schneller als in dem ersten Abflußkanal (12) strömt, daß die dispergierten Partikel an der Verzweigung (10) aufgrund der unterschiedlichen Strömungsgeschwindigkeit bevorzugt in dem zweiten Abflußkanal (12) weiterströmen.

2. Verfahren zum Abtrennen von Partikeln nach Anspruch 1 mittels eines Trennmoduls, bei welchem der erste Abflußkanal (11) zu einer weiteren Verzweigung (20) führt, so daß er einen Zuflußkanal für die weitere Verzweigung (20) bildet und von der weiteren Verzweigung ein weiterer erster Abflußkanal (21) zum Ableiten von Fluid mit verminderter Partikelkonzentration und ein weiterer zweiter Abflußkanal (22) zum Ableiten von Fluid mit erhöhter Partikelkonzentration abzweigt,
wobei das Fluid in dem weiteren zweiten Abflußkanal (22) soviel schneller als in dem weiteren ersten Abflußkanal (21) strömt, daß die dispergierten Partikel an der weiteren Verzweigung (20) aufgrund der unterschiedlichen Strömungsgeschwindigkeit bevorzugt in dem weiteren zweiten Abflußkanal (22) weiterströmen.

3. Verfahren zum Abtrennen von Partikeln nach Anspruch 2 mittels eines Trennmoduls, welcher eine Folge von Verzweigungen aufweist, deren jeweiliger Zuflußkanal von dem ersten Abflußkanal der vorausgehenden Verzweigung gebildet wird,
wobei das Fluid in dem jeweiligen zweiten von einer Verzweigung ausgehenden Abflußkanal soviel schneller als in dem jeweiligen ersten von der Verzweigung ausgehenden Abflußkanal strömt, daß die dispergierten Partikel jeweils an den Verzweigungen aufgrund der unterschiedlichen Strömungsgeschwindigkeit bevorzugt in dem jeweiligen zweiten Abflußkanal weiterströmen.

4. Verfahren zum Abtrennen von Partikeln nach Anspruch 1 mittels eines Trennmoduls, bei welchem der zweite Abflußkanal zu einer weiteren Verzweigung (25) führt, so daß er einen Zuflußkanal für die weitere Verzweigung (25) bildet und von der weiteren Verzweigung ein weiterer erster Abflußkanal (27) zum Ableiten von Fluid mit verminderter Partikelkonzentration und ein weiterer zweiter Abflußkanal (26) zum Ableiten von Fluid mit erhöhter Partikelkonzentration abzweigt,
wobei das Fluid in dem weiteren zweiten Abflußkanal (26) soviel schneller als in dem weiteren ersten Abflußkanal (27) strömt, daß die dispergierten Partikel an der weiteren Verzweigung (25) aufgrund der unterschiedlichen Strömungsgeschwindigkeit bevorzugt in dem weiteren zweiten Abflußkanal (26) weiterströmen.

5. Verfahren zum Abtrennen von Partikeln nach Anspruch 4 mittels eines Trennmoduls, welcher eine Folge von Verzweigungen (25) aufweist, deren jeweiliger Zuflußkanal (26) von dem zweiten Abflußkanal der vorausgehenden Verzweigung gebildet wird,
wobei das Fluid in dem jeweiligen zweiten von einer Verzweigung ausgehenden Abflußkanal (27) soviel schneller als in dem jeweiligen ersten von der Verzweigung ausgehenden Abflußkanal (26) strömt, daß die in dem Fluid dispergierten Partikel jeweils an den Verzweigungen aufgrund der unterschiedlichen Strömungsgeschwindigkeit bevorzugt in dem jeweiligen zweiten Abflußkanal weiterströmen.

6. Trennmodul zur Durchführung des Verfahrens nach einem der vorhergehenden Ansprüche, mit einem Substrat (5) mit Strömungskanälen (3) in Form von nutenförmigen Vertiefungen in einer Oberfläche (6) des Substrats (5), umfassend
- einen Zuflußkanal (9) zum Zuführen der Dispersion zu einer Verzweigung (10),
- einen ersten Abflußkanal (11) zum Ableiten von Fluid mit verminderter Partikelkonzentration von der Verzweigung (10) weg und
- einen zweiten Abflußkanal (12) zum Ableiten von Fluid mit erhöhter Partikelkonzentration von der Verzweigung (10) weg,
wobei die Tiefe (t) des Zuflußkanals mindestens auf dem der Verzweigung (10) unmittelbar vorausgehenden Kanalabschnitt größer als seine Breite (b) ist.

7. Trennmodul nach Anspruch 6, bei welchem die Tiefe (t) des Zuflußkanals (9) mindestens auf dem der Verzweigung (10) unmittelbar vorausgehenden Kanalabschnitt mindestens dreimal, bevorzugt mindestens fünfmal und besonders bevorzugt mindestens siebenmal so groß wie seine Breite (b) ist.

8. Trennmodul zur Durchführung des Verfahrens nach einem der vorhergehenden Ansprüche, insbesondere nach Anspruch 6 oder 7, bei welchem die Tiefe (t) des ersten Abflußkanals (11) mindestens auf dem der Verzweigung (10) unmittelbar folgenden Kanalabschnitt größer als seine Breite (b) ist.

9. Trennmodul nach Anspruch 8, bei welchem die Tiefe des (t) ersten Abflußkanals (11) mindestens auf dem der Verzweigung (10) unmittelbar folgenden Kanalabschnitt mindestens dreimal, bevorzugt mindestens fünfmal und besonders bevorzugt mindestens siebenmal so groß wie seine Breite (b) ist.

10. Trennmodul zur Durchführung des Verfahrens nach einem der Ansprüche 1 bis 5, bei welchem die kleinste Querschnittsdimension des ersten Abflußkanals (11) mindestens 5 *µ*m und höchstens 150 *µ*m beträgt.

11. Trennmodul zur Durchführung des Verfahrens nach einem der Ansprüche 1 bis 5, bei welchem der Strömungswiderstand des ersten Abflußkanals (11) höher als der Strömungswiderstand des zweiten Abflußkanals (12) ist.

12. Trennmodul nach Anspruch 11, bei welchem der höhere Strömungswiderstand des ersten Abflußkanals (11) daraus resultiert, daß er länger als der zweite Abflußkanal (12) ist.

13. Trennmodul zur Durchführung des Verfahrens nach einem der Ansprüche 1 bis 5, bei welchem der Zuflußkanal (9) und der erste erste Abflußkanal (11) in ihrem an die Verzweigung (10) angrenzenden Kanalabschnitt die gleiche Tiefe (t) haben.

## Claims

1. Method for separating particles from a fluid dispersion, particularly for separating corpuscular components from biological samples, above all from blood,
by means of a separating module comprising a substrate (5) with flow channels (3) forming groove-shaped recesses in a surface (6) of the substrate (5), including
- a feed channel (9) for supplying the dispersion to a junction (10),
- a first drain channel (11) for draining fluid having a reduced particle concentration away from the junction (10), and
- a second drain channel (12) for draining fluid having an increased particle concentration away from the junction (10),
wherein the fluid flows so much faster in the second drain channel than in the first drain channel (12) that due to the different flow speeds the dispersed particles at the junction (10) preferentially flow further in the second drain channel (12).

2. Method for separating particles according to claim 1 by means of a separating module, wherein the first drain channel (11) leads to a further junction (20), so that it forms a feed channel for the further junction (20) and a further first drain channel (21) for draining fluid having a reduced particle concentration and a further second drain channel (22) for draining fluid having an increased particle concentration branch off from the further junction,
wherein the fluid flows so much faster in the further second drain channel (22) than in the further first drain channel (21) that due to the different flow speeds the dispersed particles preferentially flow at the further junction (20) into the further second drain channel (22) .

3. Method for separating particles according to claim 2 by means of a separating module which has a sequence of junctions, each having a feed channel formed by the first drain channel of the preceding junction,
wherein the fluid flows so much faster in the particular second drain channel originating from a junction than in the particular first drain channel originating from the junction that due to the different flow speeds the dispersed particles preferentially flow at each of the junctions further in the particular second drain channel.

4. Method for separating particles according to claim 1 by means of a separating module, wherein the second drain channel leads to a further junction (25), so that it forms a feed channel for the further junction (25), and a further first drain channel (27) branches off from the further junction for draining fluid having a reduced particle concentration, and a further second drain channel (26) branches off from the further junction for draining fluid having an increased particle concentration,
wherein the fluid flows so much faster in the further second drain channel (26) than in the further first drain channel (27) that due to the different flow speeds the dispersed particles preferentially flow at the further junction (25) further in the further second drain channel (26).

5. Method for separating particles according to claim 4 by means of a separating module which has a sequence of junctions (25), each having a feed channel (26) formed by the second drain channel of the preceding junction,
wherein the fluid flows so much faster in the particular second drain channel (27) originating from a junction than in the particular first drain channel (26) originating from the junction that due to the different flow speeds the particles dispersed in the fluid preferentially flow at each of the branches further in the particular second drain channel.

6. Separating module for performing the method according to one of the preceding claims, comprising a substrate (5) with flow channels (3) forming groove-shaped recesses in a surface (6) of the substrate (5), including
- a feed channel (9) for supplying the dispersion to a junction (10),
- a first drain channel (11) for draining fluid having a reduced particle concentration away from the junction (10), and
- a second drain channel (12) for draining fluid having an increased particle concentration away from the junction (10),
wherein the depth (t) of the feed channel is greater than its width (b), at least in the channel section immediately preceding the junction (10).

7. Separating module according to claim 6, wherein the depth (t) of the feed channel (9) is at least three times, preferably at least five times, and especially preferably at least seven times as large as its width (b), at least in the channel section immediately preceding the junction (10).

8. Separating module for performing the method according to one of the preceding claims, in particular according to claim 6 or 7, wherein the depth (t) of the first drain channel (11) is greater than its width (b), at least in the channel section immediately following the junction (10).

9. Separating module according to claim 8, wherein the depth (t) of the first drain channel (11) is at least three times, preferably at least five times, and especially preferably at least seven times as large as its width (b), at least in the channel section immediately following the junction (10).

10. Separating module for performing the method according to any one of claims 1 to 5, wherein the smallest cross-sectional dimension of the first drain channel (11) is at least 5 µm and at most 150 µm.

11. Separating module for performing the method according to any one of claims 1 to 5, wherein the flow resistance of the first drain channel (11) is higher than the flow resistance of the second drain channel (12).

12. Separating module according to claim 11, wherein the higher flow resistance of the first drain channel (11) results from the fact that the first drain channel (11) is longer than the second drain channel (12).

13. Separating module for performing the method according to any one of claims 1 to 5, wherein the feed channel (9) and the first drain channel (11) have equal depths (t) in their channel sections adjoining the junction (10).

## Revendications

1. Procédé pour la séparation de particules d'une dispersion fluide, notamment pour la séparation d'éléments corpusculaires provenant d'échantillons biologiques, avant tout du sang, au moyen d'un module de séparation doté d'un substrat (5) comprenant des canaux d'écoulement (3) sous forme de creux en forme de rainure dans une surface (6) du substrat (5), comportant
- un canal d'alimentation (9) destiné à amener la dispersion à une ramification (10),
- un premier canal d'évacuation (11) destiné à évacuer de la ramification (10) le fluide comprenant une concentration de particules moins élevée et
- un second canal d'évacuation (12) destiné à évacuer de la bifurcation (10) le fluide comprenant une concentration de particules plus élevée,
le fluide s'écoulant tellement plus rapidement dans le second canal d'évacuation que dans le premier canal d'évacuation (12) que les particules dispersées au niveau de la ramification (10) continuent de s'écouler de préférence dans le second canal d'évacuation (12) en raison de la différence de vitesse d'écoulement.

2. Procédé pour la séparation des particules selon la revendication 1 au moyen d'un module de séparation, dans lequel le premier canal d'évacuation (11) mène à une autre ramification (20), de sorte qu'il forme un canal d'alimentation pour l'autre ramification (20) et de l'autre ramification dérivent un autre premier canal d'évacuation (21) destiné à évacuer le fluide comprenant une concentration de particules moins élevée et un autre second canal d'évacuation (22) destiné à évacuer le fluide comprenant une concentration de particules plus élevée, le fluide s'écoulant tellement plus rapidement dans l'autre second canal d'évacuation (22) que dans l'autre premier canal d'évacuation (21) que les particules dispersées au niveau de l'autre ramification (20) continuent de s'écouler de préférence dans l'autre second canal d'évacuation (22) en raison de la différence de vitesse d'écoulement.

3. Procédé pour la séparation des particules selon la revendication 2 au moyen d'un module de séparation, lequel comprend une succession de ramifications, dont le canal d'alimentation respectif est formé par le premier canal d'évacuation de la ramification précédente, le fluide s'écoulant tellement plus rapidement dans le second canal d'évacuation respectif partant d'une ramification que dans le premier canal d'évacuation respectif partant de la ramification que les particules dispersées respectivement au niveau des ramifications continuent de s'écouler de préférence dans le second canal d'évacuation respectif en raison de la différence de vitesse d'écoulement.

4. Procédé pour la séparation des particules selon la revendication 1 au moyen d'un module de séparation, dans lequel le second canal d'évacuation mène à une autre ramification (25), de sorte qu'il forme un canal d'alimentation pour l'autre ramification (25) et que de l'autre ramification dérivent un autre premier canal d'évacuation (27) destiné à évacuer le fluide comprenant une concentration de particules moins élevée et un autre second canal d'évacuation (26) destiné à évacuer le fluide comprenant une concentration de particules plus élevée, le fluide s'écoulant tellement plus rapidement dans l'autre second canal d'évacuation (26) que dans l'autre premier canal d'évacuation (27) que les particules dispersées au niveau de l'autre ramification (25) continuent de s'écouler de préférence dans l'autre second canal d'évacuation (26) en raison de la différence de vitesse d'écoulement.

5. Procédé pour la séparation des particules selon la revendication 4 au moyen d'un module de séparation, lequel comprend une succession de ramifications (25), dont le canal d'alimentation (26) respectif est formé par le second canal d'évacuation de la ramification précédente, le fluide s'écoulant tellement plus rapidement dans le second canal d'évacuation (27) respectif partant d'une ramification que dans le premier canal d'évacuation (26) respectif partant de la ramification que les particules dispersées respectivement au niveau des ramifications continuent de s'écouler de préférence dans le second canal d'évacuation respectif en raison de la différence de vitesse d'écoulement.

6. Module de séparation pour mettre en oeuvre le procédé selon l'une quelconque des revendications précédentes, doté d'un substrat (5) comprenant des canaux d'écoulement (3) sous forme de creux en forme de rainure dans une surface (6) du substrat (5), comportant
- un canal d'alimentation (9) destiné à amener la dispersion à une ramification (10),
- un premier canal d'évacuation (11) destiné à évacuer de la ramification (10) le fluide comprenant une concentration de particules moins élevée et
- un second canal d'évacuation (12) destiné à évacuer de la bifurcation (10) le fluide comprenant une concentration de particules plus élevée,
la profondeur (t) du canal d'alimentation, au moins sur la section transversale de canal précédant immédiatement la ramification (10), étant supérieure à sa largeur (b).

7. Module de séparation selon la revendication 6, dans lequel la profondeur (t) du canal d'alimentation (9), au moins sur la section transversale de canal précédant immédiatement la ramification (10), est au moins trois fois, de préférence au moins cinq fois et de manière préférée entre toutes au moins sept fois supérieure à sa largeur (b).

8. Module de séparation destiné à réaliser le procédé selon l'une quelconque des revendications précédentes, notamment selon la revendication 6 ou 7, dans lequel la profondeur (t) du premier canal d'évacuation (11), au moins sur la section transversale de canal suivant immédiatement la ramification (10), est supérieure à sa largeur (b).

9. Module de séparation selon la revendication 8, dans lequel la profondeur (t) du premier canal d'évacuation (11), au moins sur la section transversale de canal suivant immédiatement la ramification (10), est au moins trois fois, de préférence au moins cinq fois et de manière préférée entre toutes au moins sept fois supérieure à sa largeur (b).

10. Module de séparation pour mettre en oeuvre le procédé selon l'une quelconque des revendications 1 à 5, dans lequel la plus petite dimension en section transversale du premier canal d'évacuation (11) mesure au moins 5 µm et au maximum 150 µm.

11. Module de séparation pour mettre en oeuvre le procédé selon l'une quelconque des revendications 1 à 5, dans lequel la résistance à l'écoulement du premier canal d'évacuation (11) est supérieure à la résistance à l'écoulement du second canal d'évacuation (12).

12. Module de séparation selon la revendication 11, dans lequel la résistance à l'écoulement plus élevée du premier canal d'évacuation (11) résulte du fait que ledit canal est plus long que le second canal d'évacuation (12).

13. Module de séparation pour mettre en oeuvre le procédé selon l'une quelconque des revendications 1 à 5, dans lequel le canal d'alimentation (9) et le premier canal d'évacuation (11) présentent dans leur section transversale de canal adjacente à la ramification (10) la même profondeur (t).
